(19) Europäisches Patentamt
European Patent Office
Office européen des brevets

(11) Veröffentlichungsnummer: **0 193 770**
**A2**

(12) **EUROPÄISCHE PATENTANMELDUNG**

(21) Anmeldenummer: **86101966.9**

(22) Anmeldetag: **17.02.86**

(51) Int. Cl.⁴: **C 07 D 211/46**, A 61 K 31/445

(30) Priorität: **28.02.85 DE 3507019**

(43) Veröffentlichungstag der Anmeldung: **10.09.86 Patentblatt 86/37**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Kinast, Günther, Dr., Eckbusch 15A, D-5600 Wuppertal 1 (DE)**

(54) **Neue Derivate von 3,4,5-Trihydroxypiperidin, Verfahren zu ihrer Herstellung und ihre Verwendung.**

(57) Die Erfindung betrifft neue Derivate von 3,4,5-Trihydroxypiperidin, Verfahren zu ihrer Herstellung sowie ihre Verwendung als Mannosidaseinhibitoren.

Die neuen Derivate lassen sich durch die Formel (I) wiedergeben

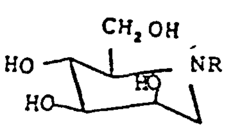
(I)

in der R einen gegebenenfalls substituierten, geradkettigen, verzweigten oder cyclischen gesättigten oder ungesättigten aliphatischen Kohlenwasserstoffrest darstellt.

EP 0 193 770 A2

BAYER AKTIENGESELLSCHAFT 509 Leverkusen-Bayerwerk
Konzernverwaltung RP
Patentabteilung Ad/li-c

# Neue Derivate von 3,4,5-Trihydroxypiperidin, Verfahren zu ihrer Herstellung und ihre Verwendung

Die vorliegende Erfindung betrifft neue Derivate von 3,4,5-Trihydroxypiperidin, Verfahren zu ihrer Herstellung sowie ihre Verwendung als Mannosidaseinhibitoren.

Die neuen Derivate lassen sich durch die Formel (I) wiedergeben

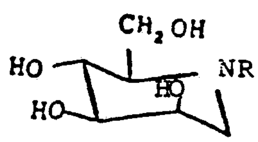

(I)

in der

R einen gegebenenfalls substituierten, geradkettigen, verzweigten oder cyclischen gesättigten oder ungesättigten aliphatischen Kohlenwasserstoffrest darstellt.

Die Erfindung betrifft darüberhinaus auch pharmazeutisch verwendbare Hydro-Salze der Verbindungen der Formel (I), wie Hydrochloride, -sulfate, -acetate, -carbonate, -oxalate usw., und Bio-Vorläufer, wobei unter Bio-Vorläufer

Verbindungen verstanden werden, deren Struktur sich von der aktiven Verbindung unterscheidet, die jedoch nach Verabreichung an Mensch oder Tier im Körper des Patienten in die aktive Verbindung umgewandelt werden.

Bevorzugt bedeutet R einen Alkylrest mit 1 bis 30, insbesondere 1 bis 18 C-Atomen, einen Alkenylrest oder Alkinylrest mit 2 bis 18, insbesondere 3 bis 10 C-Atomen.

Die genannten Alkyl, Alkenyl und Alkinylreste können gradkettig, verzweigt oder cyclisch und durch O und S unterbrochen und durch folgende Reste substituiert sein:

Hydroxy, Alkoxy, Aryloxy, Amino, substituierte Amino wie z. B. Phthalimido, Alkylthio, Arylthio, substituiertes Aryl, Heteroaryl, Halogen wie Fluor, Chlor und Brom, Carboxy, Carboxyalkyl sowie Nitro.

Es wurde gefunden, daß die Verbindungen der Formel I potente Inhibitoren zellulärer Mannosidasen sind und damit wertvolle Mittel zur Beeinflussung der Biosynthese der Glycoproteine sind.

Die Verbindungen der Formel I erhält man, indem man das Manno-1-desoxyisonojirimycins (II) (Kinast, Schedel, Angew. Chem. Int. Ed. Engl. 20 (1981), 805-6) am Stickstoff alkyliert. Dazu sind bekannte N-Alkylierungsmethoden geeignet, z. B. die reduktive Alkylierung mit Carbonylverbindungen und Wasserstoff-Donator-Reduktionsmitteln, die direkte Alkylierung mit Alkylhalogeniden und die Reduktion von Acylverbindungen.

Beispielhaft seien folgende Methoden aufgeführt:

1. Reduktive Alkylierung.
Hierbei werden Verbindungen der Formel (II) mit einem Keton oder Aldehyd der allgemeinen Formel (III)

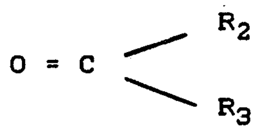

(III)

in der
$R^2$ und $R^3$ entweder H bedeuten oder die oben für R angegebene Bedeutung besitzen oder $R^2$ für H und $R^3$ für die oben angegebene Bedeutung von R steht, und einem geeigneten Reduktionsmittel wie z. B. Natriumcyanoborhydrid, Natriumborhydrid/Trifluoressigsäure oder Raney-Nickel/Wasserstoff nach dem folgenden Schema umgesetzt.

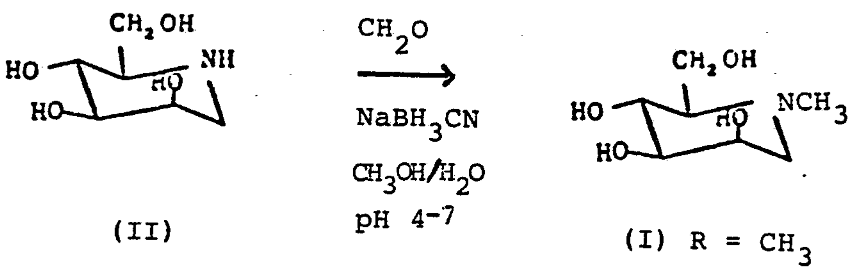


(II) (I) R = $CH_3$

2. Alkylierung.
Zur direkten Alkylierung werden Verbindungen der Formel (II) in einem geeigneten Lösemittel wie z. B. Dimethylformamid mit einem Alkylierungsmittel

der allgemeinen Formel (IV)

Z - R (IV)

in der
R die oben angegebene Bedeutung besitzt und Z eine bei einem Alkylierungsmittel gebräuchliche leicht austretende Gruppe darstellt, z. B. Halogen, insbesondere Chlor, Brom oder Jod, und einer geeigneten Base wie z. B. $K_2CO_3$ nach folgendem Schema umgesetzt

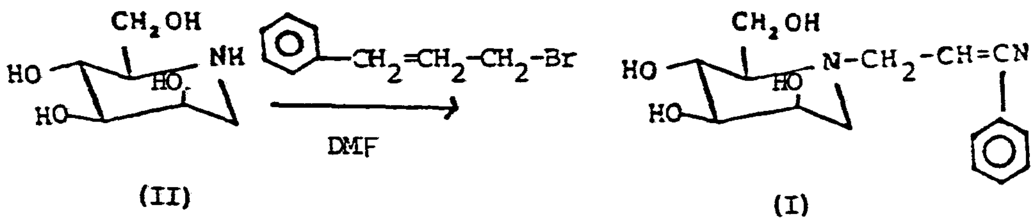


(II) (I)

3. Reduktion von Acylverbindungen.
Bei diesem Verfahren zur Herstellung der Verbindungen der Formel I wird (II) zuerst in Wasser, Wasser/Alkoholgemischen oder z. B. in Dimethylformamid (DMF) mit einem Acylhalogenid der allgemeinen Formel (V)

$R^2$-CO-Hal

in der
R die oben angegebene Bedeutung besitzt und Hal für Halogen, insbesondere Chlor, steht, oder einem entsprechenden Anhydrid zu (VI) acyliert. Anschließend wird (VI) z. B. mit $LiAlH_4$ zu (I) reduziert.

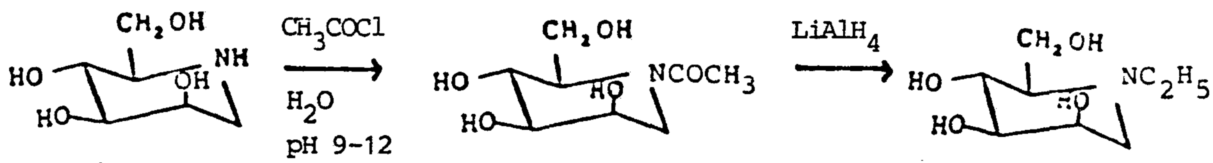


(II) (VI) (I) $R=C_2H_5$

Die erfindungsgemäß verwendeten Carbonylverbindungen für die reduktive Alkylierung sind entweder bekannt oder können nach Standardverfahren hergestellt werden.

Als typische Beispiele seien im einzelnen genannt:

Gerad- oder verzweigtkettige Alkylaldehyde wie Formaldehyd, Acetaldehyd, n-Propanal, n-Butanal, 2-Methylpropanal, n-Pentanal, 2-Methylbutanal, 3-Methylbutanal, 2,2-Dimethylpropanal, n-Hexanal, 2-Äthylbutanal, n-Heptanal und n-Octanal; Alkenylaldehyde wie Propenal, 2-Methylpropenal, 2-Butenal, 2-Methyl-2-butenal, 2-Äthyl-2-hexenal; cyclische Aldehyds wie Cyclopentancarbaldehyd, Cyclopentanacetaldehyd, Cyclohexancarbaldehyd; Benzaldehyd, o-, m- und p-Toluolcarbaldehyde und Phenylacetaldehyd; durch Hydroxy substituierte gerad- und verzweigtkettige Alkylaldehyde wie 5-Hydroxypentanal, 2-Hydroxy-3-methylbutanal, 2-Hydroxy-2-methylpropanal, 4-Hydroxybutanal, 2-Hydroxypropanal und 8-Hydroxyoctanal; durch Alkylamino substituierte gerad- und verzweigtkettige Alkylaldehyde wie 5-Dimethylaminopentanal, 2-Diethylaminopropanal.

Des weiteren:

Methoxy-acetaldehyd, Aethoxy-acetaldehyd, n-Propoxy-acetaldehyd, i-Propoxy-acetaldehyd, n-Butoxy-acetaldehyd, i-Butoxy-acetaldehyd, tert.-Butoxy-acetaldehyd, Cyclopropylmethyloxy-acetaldehyd, Cyclopropoxy-acetaldehyd, 2-Methoxy-äthoxy-acetaldehyd, 2-Aethoxy-äthoxy-acetaldehyd, 2-Methoxy(1-methyl-äthoxy)-acetaldehyd, 2-Aethoxy(1-methyl-äthoxy)-acetaldehyd, Phenyloxy-acetaldehyd, 2-Methoxy-2-methyl-acetaldehyd, 2-Aethoxy-2-methyl-acetaldehyd, 2-n-Propoxy-2-methyl-acetaldehyd, 2-(i-Propoxy-)2-methyl-acetaldehyd, 2-(n-Butoxy)-2-methyl-acetaldehyd, 2-(i-Butoxy)-2-methyl-acetaldehyd, 2-(tert.-Butoxy)-2-methyl-acetaldehyd, 2-Cyclopropylmethyloxy-2-methyl- acetaldehyd, 2-Cyclopropyloxy-2-methyl-acetaldehyd, 2-Methoxy-äthoxy-2-methyl-acetaldehyd, 2-Aethoxy-äthoxy-2-methyl-acetaldehyd, 2-Methoxy-(1-methyl-äthoxy)2-methyl-acetaldehyd, 2-Methoxy-2,2-dimethyl-acetaldehyd, 2-Aethoxy-2,2-dimethyl-acetaldehyd, 2-Cyclopropylmethyloxy-acetaldehyd, 2-Butoxy-2,2-dimethyl-acetaldehyd, Methylthio-acetaldehyd, Aethylthio-acetaldehyd, n-Propylthio-acetaldehyd, i-Propylthio-acetaldehyd, Cyclopropyl-methylthio-acetaldehyd, 3-Methoxypropanal, 3-Aethoxy-propanal, 3-n- und 3-i-Propoxypropanal, 3-n-, 3-i- und 3-tert.-Butoxy-propanal, 3-Cyclopropyloxy-propanal, 3-Cyclopropylmethyloxy-propanal, 3-Methoxy-3-methyl-propanal, 3-Aethoxy-3-methyl-propanal, 3-n- und 3-i-propoxy-3-methyl-propanal, 3-n-, 3-i- und 3-tert.-Butoxy-3-methyl-propanal, 2,3 und 4-Methoxy-butanal, 2,3 und 4-aethoxy-

butanal, 2-Methylthio-propanal, 2-Aethylthio-propanal, 3-Methylthio-propanal, 3-Aethylthio-propanal, 2-Methylthio-Butanal, 3-Methylthio-butanal, 4-Methylthio-butanal, Furfurol, Tetrahydrofurfurol, Thiophen, 5-Bromthiophen, 5-Methylfurfurol, Pyran-carbaldehyd.

Außerdem seien als Ketone beispielsweise genannt:

Aceton, Methyläthylketon, Methyl-n-propylketon, Diäthylketon, Methylbutylketon, Cyclopentanon, Di-n-propyl-keton, Cyclohexanon, 3-Methylcyclohexanon, 4-Methylcyclohexanon, Acetophenon, Propiophenon, Butyrophenon, Phenylaceton, p-Methoxyacetophenon, m-Nitroacetophenon.

Als Wasserstoff-Donor-Reduktionsmittel für die reduktive Alkylierung kann man beispielsweise Ameisensäure verwenden (Leuckart-Wallach-Reaktion). Die Ameisensäure wird in großem Überschluß verwendet. Mit Formaldehyd als Carbonylkomponente kann die Reaktion in wäßriger Lösung durchgeführt werden, mit Ketonen und weniger reaktionsfähigen Aldehyden in wasserfreier Ameisensäure. Die Reaktionstemperaturen liegen zwischen 100 und 200°C, gegebenenfalls muß die Reaktion in einem Autoklaven durchgeführt werden.

Als Wasserstoff-Donor-Reduktionsmittel kann man auch katalytisch erregten Wasserstoff verwenden. Als Katalysator kommt vor allem Raney-Nickel in Frage, es können aber auch Edelmetallkatalysatoren Verwendung finden. Die Reaktion wird im allgemeinen bei Drucken zwischen 80 und 150 Atmosphären $H_2$-Druck und Temperaturen zwischen 70

und 150°C durchgeführt. Als Lösungsmittel werden protische, polare Lösungsmittel besonders Alkohole bevorzugt.

Als Wasserstoff-Donor-Reduktionsmittel werden auch Alkalimetallcyanoborhydride, Dialkylaminoborane und Alkalimetallborhydride verwendet. Besonders bevorzugt in dieser Verfahrensvariante ist die Verwendung von Natriumcyanoborhydrid.

Die Reaktion wird im allgemeinen bei Raumtemperatur durchgeführt. Es kann aber auch günstig sein, auf Rückflußtemperatur zu erhitzen.

Das Verfahren wird üblicherweise in einem inerten Lösungsmittel durchgeführt. Obwohl wasserfreie aprotische Lösungsmittel eingesetzt werden können (z. B. Tetrahydrofuran, wenn das Reduktionsmittel Morpholinoboran ist), wird gewöhnlich doch ein protisches Lösungsmittel verwendet. Als solches eignet sich besonders ein niederes Alkanol. Es kann aber auch Wasser oder ein wäßriges niedriges Alkanol (z. B. wäßriges Methanol oder Aethanol) oder andere wäßrige Lösungsmittelsysteme, wie z. B. wäßriges Dimethylformamid, wäßriges Hexamethylphosphorsäuretriamid, wäßriges Tetrahydrofuran oder wäßriger Aethylenglycoldimethyläther verwendet werden.

Das Verfahren wird gewöhnlich in einem pH-Bereich von 1 bis 11 durchgeführt, bevorzugt ist ein pH-Bereich zwischen 4 und 7.

Als neue Wirkstoffe seien im einzelnen aufgeführt die Verbindungen der Formel (I)

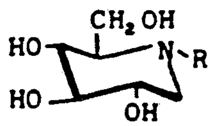

(I)

| mit | R |
|---|---|
| | $CH_3$ - |
| | $CH_3CH_2$- |
| | $CH_3CH_2CH_2$- |
| | $CH_3CHCH_3$ |
| | $CH_3CH_2CH_2CH_2$- |
| | 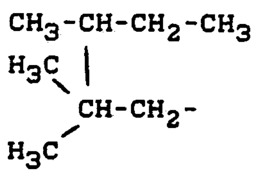 |
| | 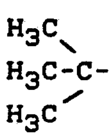 |
| | $CH_3(CH_2)_3$-$CH_2$- |

| R |
|---|
| 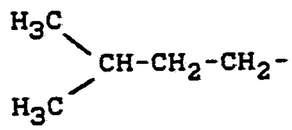 |
| $CH_3$- $\underset{\vert}{C}HCH_2CH_2CH_3$ |
| $CH_3CH_2$-$\underset{\vert}{C}HCH_2CH_3$ |
| $CH_3\underset{\vert}{C}HCH_2CH_2$- <br> $CH_3$ |
| $CH_3$ $(CH_2)_4$-$CH_2$- |
| $CH_3$ $(CH_2)_5$-$CH_2$- |
| $CH_3\underset{\vert}{C}HCH_2CH_2CH_2$- <br> $CH_3$ |
| $CH_3\underset{\vert}{C}H$-$(CH_2)_3$-$CH_2$- <br> $CH_3$ |
| $CH_3(CH_2)_6$-$CH_2$- |
| $CH_3\underset{\vert}{C}H$-$(CH_2)_4$-$CH_2$- <br> $CH_3$ |
| $CH_3$-$(CH_2)_8$-$CH_2$- |
| $CH_3$-$(CH_2)_{10}$-$CH_2$- |
| $CH_3$-$(CH_2)_{12}$-$CH_2$- |

R

$CH_3-(CH_2)_{14}-CH_2-$

$CH_3-(CH_2)_{16}-CH_2-$

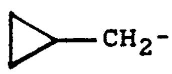

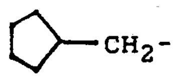

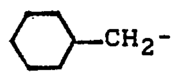

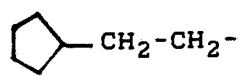

$HO-CH_2-CH_2-$

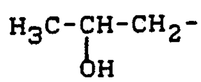

$HOH_2C-CH_2-CH_2-$

$HOH_2C-CH_2-CH_2-CH_2-$

$HOH_2C-(CH_2)_3-CH_2-$

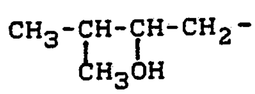

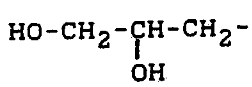

R

$CH_3OCH_2-CH_2-$

$C_3H_7OCH_2-CH_2-$

$CH_3COOCH_2-CH_2-$

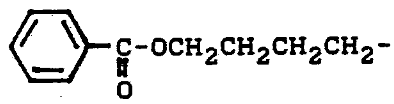

$H_2N-CH_2-CH_2-$

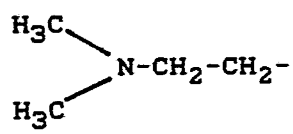

$CH_3CONH-CH_2-CH_2-$

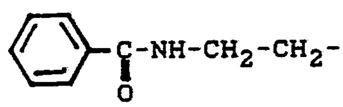

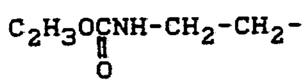

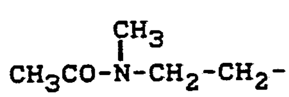

$CH_3NH-CO-NH-CH_2CH_2-$

| R |
| --- |
| 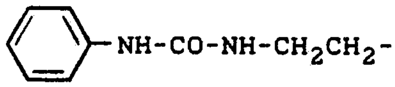 |
| $CH_3NH-CS-NH-CH_2CH_2-$ |
| 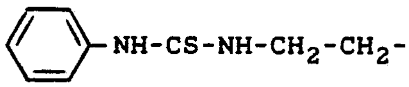 |
| $H_2N-CH_2CH_2CH_2-$ |
| $CH_3CONHCH_2CH_2CH_2-$ |
| 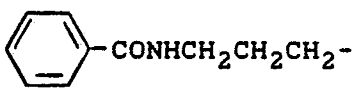 |
| $CH_3NHCONHCH_2CH_2CH_2-$ |
| 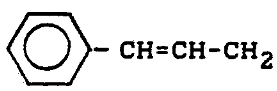 |
| 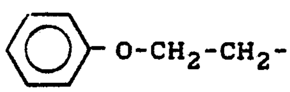 |
| 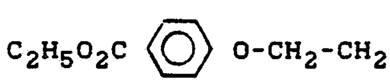 |

| R |
| --- |
| 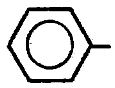 $-CH_2-O-CH_2-CH_2-$ |
| 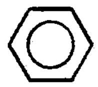 $-O-CH_2-CH_2-$ |
| 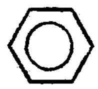 $-O_2C-CH_2-CH_2-CH_2-$ |
| $H_2N-CH_2CH_2CH_2CH_2-$ |
| $H_2C=CH-CH_2-$ |
| $H_3CHC=CH-CH_2$ |
| $H_2C=CH-CH_2-CH_2-$ |
| $H_2C=CH-CH_2-CH_2-CH_2-CH_2-$ |
| $H_2C=CH-(CH_2)_7-CH_2-$ |
| $HOOC-CH_2-$ |
| $HOOC-CH_2-CH_2-$ |
| $H_3C_2OOC-CH_2-CH_2-$ |
| $H_2N-C(=O)-CH_2-$ |
| $C_2H_3HN-C(=O)-CH_2$ |

| R |
|---|
| $C_4H_9-HN-\underset{\underset{O}{\Vert}}{C}-CH_2-$ |
| $HO_3S-CH_2CH_2CH_2-$ |
| $H_2NO_2S-CH_2CH_2CH_2-$ |
| 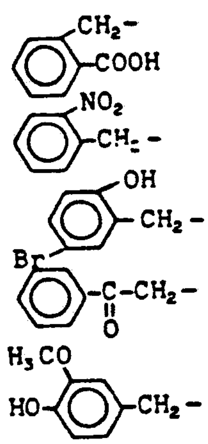 |
| $H-C\equiv C-CH_2-$ |

R

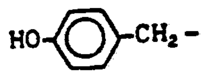

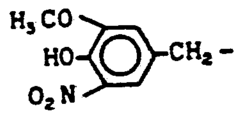

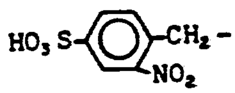

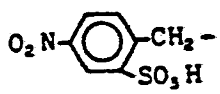

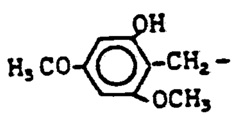

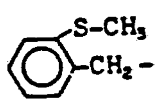

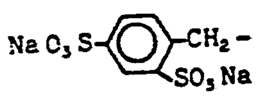

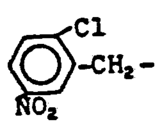

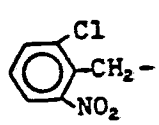

R

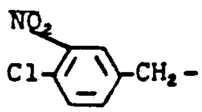

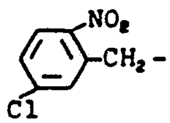

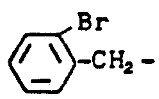

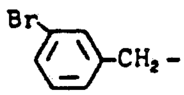

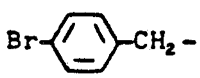

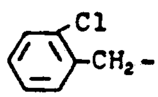

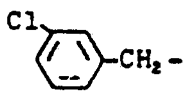

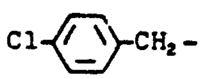

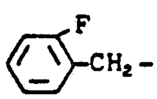

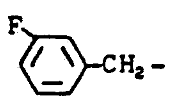

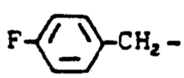

R

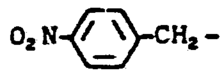

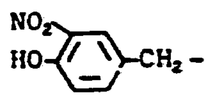

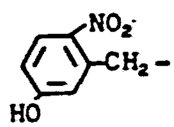

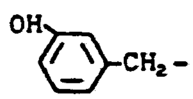

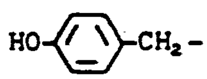

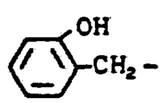

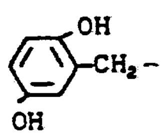

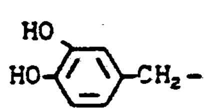

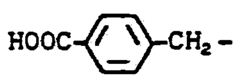

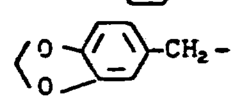

R

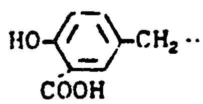

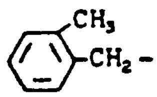

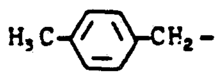

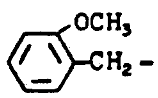

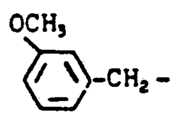

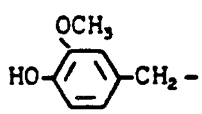

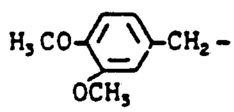

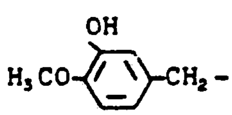

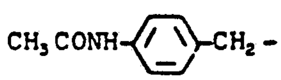

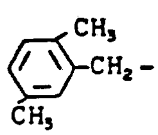

R

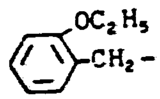

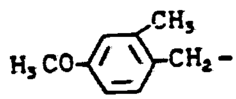

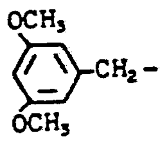

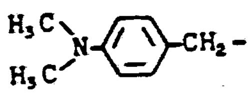

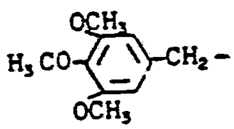

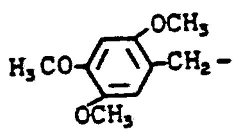

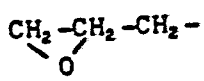

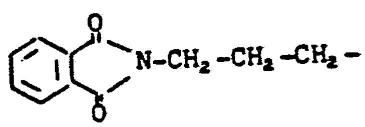

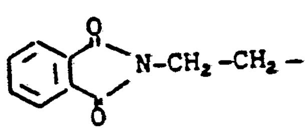

R

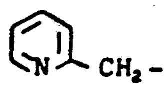

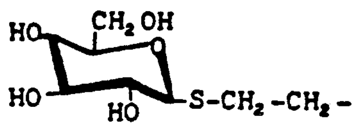

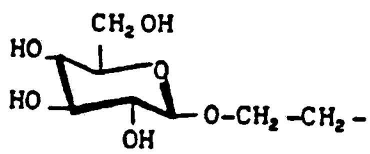

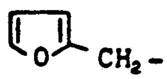

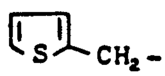

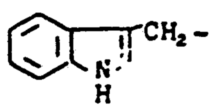

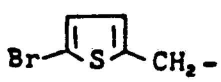

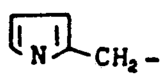

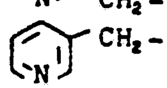

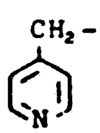

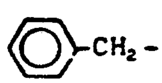

Die erfindungsgemäßen Inhibitoren eignen sich als Mannosidase-Inhibitoren.

Zur Verbreiterung des Wirkungsspektrums kann es sich empfehlen, Inhibitoren für Glycosidhydrolasen, die sich gegenseitig in ihrer Wirkung ergänzen, zu kombinieren, sei es, daß es sich um Kombinationen der erfindungsgemäßen Inhibitoren untereinander oder um Kombinationen der erfindungsgemäßen Inhibitoren mit bereits bekannten handelt. So kann es beispielsweise zweckmäßig sein, erfindungsgemäße Mannosidase-Inhibitoren mit bereits bekannten Amylase-Inhibitoren zu kombinieren.

Pharmazeutische Zubereitungen können eine größere oder kleinere Menge des Inhibitors enthalten, z.B. 0,1 % bis 99,5 %, in Kombination mit einem pharmazeutisch verträglichen nichttoxischen, inerten Trägerstoff, wobei der Trägerstoff eine oder mehrere feste, halbfeste oder flüssige Verdünnungsmittel, Füllstoffe und/oder nichttoxisches, inertes und pharmazeutisch-verträgliches Formulierungshilfsmittel enthalten kann. Solche pharmazeutischen Zubereitungen liegen vorzugsweise in Form von Dosierungseinheiten vor, d. h. physikalisch-diskrete, eine bestimmte Menge des Inhibitors enthaltenden Einheiten, die einem Bruchteil oder einem Vielfachen der Dosis entsprechen, die zur Herbeiführung der gewünschten Hemmwirkung entsprechen. Die Dosierungseinheiten können 1, 2, 3, 4 oder mehr Einzeldosen oder 1/2, 1/3 oder 1/4 einer Einzeldosis enthalten. Eine Einzeldosis enthält vorzugsweise eine genügende Menge Wirkstoff, um

bei einer Applikation gemäß eines vorher bestimmten Dosierungsschemas einer oder mehrerer Dosierungseinheiten die gewünschte Hemmwirkung zu erzielen, wobei eine ganze, eine halbe, oder ein Drittel oder ein Viertel der Tagesdosis gewöhnlich zu allen Haupt- und Nebenmahlzeiten am Tage verabreicht wird. Andere therapeutische Mittel können auch eingenommen werden. Obgleich die Dosierung und das Dosierungsschema in jedem Fall sorgsam abgewogen werden sollte, unter Anwendung gründlichen fachmännischen Urteils und unter Beachtung des Alters, des Gewichts und des Zustands des Patienten, der Art und der Schwere der Erkrankung, wird die Dosierung gewöhnlich in einem Bereich zwischen etwa 1 bis etwa $1 \times 10^4$ SIE/kg des Körpergewichtes pro Tag liegen. In manchen Fällen wird man dabei eine ausreichende therapeutische Wirkung mit einer geringeren Dosis erreichen, während in anderen Fällen eine größere Dosis erforderlich sein wird.

Orale Applikation kann unter Verwendung fester und flüssiger Dosierungseinheiten durchgeführt werden, wie z. B. Pulver, Tabletten, Dragees, Kapseln, Granulate, Suspensionen, Lösungen und dergleichen.

Pulver wird durch Zerkleinerung der Substanz in einer geeigneten Größe und Vermischen mit einem ebenfalls zerkleinerten pharmazeutischen Trägerstoff hergestellt. Obgleich ein eßbares Kohlenhydrat, wie z. B. Stärke, Lactose, Saccharose oder Glucose normalerweise zu diesem Zwecke Verwendung findet und auch hier benutzt werden kann, ist es wünschenswert ein nicht metabolisierbares Kohlenhydrat, wie z. B. ein Cellulosederivat zu benutzen.

Süßmittel, Geschmackszusätze, Konservierungsstoffe, Dispergiermittel und Färbemittel können auch mitverwendet werden.

Die Kapseln können durch Zubereitung der oben beschriebenen Pulvermischung und durch Füllung bereits gebildeter Gelatinehüllen hergestellt werden. Die Pulvermischung kann man vor dem Füllvorgang mit Gleitmitteln, wie z. B. Kieselgel, Talkum, Magnesiumstearat, Calciumstearat oder festem Polyäthylenglykol versetzen. Die Mischung kann man ebenfalls mit einem Desintegrator oder Lösungsvermittler, wie z. B. Agar-Agar, Calciumcarbonat oder Natriumcarbonat versetzen, um bei Einnahme der Kapsel die Zugänglichkeit des Inhibitors zu verbessern.

Die Anfertigung der Tabletten erfolgt zum Beispiel durch Herstellung einer Pulvermischung, grob oder feinkörnig, und Hinzufügung eines Gleitmittels und Desintegrators. Aus dieser Mischung formt man Tabletten. Ein Pulvermischung bereitet man vor durch Mischung der Substanz, welche in geeigneter Weise zerkleinert wurde und ergänzt ein Verdünnungsmittel oder eine andere Trägersubstanz wie oben beschrieben. Gegebenenfalls fügt man ein Bindemittel hinzu: z. B. Carboxymethylcellulose, Alginate, Gelatine oder Polyvinylpyrrolidone, einen Lösungsverlängerer, wie z. B. Paraffin, einen Resorptionsbeschleuniger, wie z. B. ein quarternäres Salz und/oder ein Adsorptionsmittel, wie z. B. Bentonit, Kaolin oder Dicalciumphosphat. Die Pulvermischung kann granuliert werden zusammen mit einem Bindemittel, wie z. B. Sirup, Stärkepaste, Akazienschleim, oder Lösungen aus Zellulose- oder Polymerenmaterialien. Danach preßt man das

Produkt durch ein grobes Sieb. Als Alternative hierzu kann man die Pulvermischung durch eine Tablettenmaschine laufen lassen und die sich ergebenden ungleichmäßig geformten Stücke bis auf Korngröße zerkleinern. Damit die entstandenen Körner nicht in den tablettenbildenden Düsen stecken bleiben, kann man sie mit einem Gleitmittel versetzen, wie z. B. Stearinsäure, Stearatsalz, Talkum oder Mineralöl. Diese gleitfähig gemachte Mischung wird dann in Tablettenform gepreßt. Die Wirkstoffe können auch mit freifließenden inerten Trägerstoffen vereinigt werden und direkt in Tablettenform gebracht werden unter Auslassung der Granulat- oder Zerstückelungsschritte. Man kann das Produkt mit einer klaren oder opaken Schutzhülle versehen, z. B. einem Überzug aus Schellack, einem Überzug aus Zucker oder Polymersubstanzen und einer polierten Hülle aus Wachs. Farbstoffe können diesen Überzügen beigefügt werden, damit zwischen den verschiedenen Dosierungseinheiten unterschieden werden kann.

Die oral zu verabreichenden Zubereitungsformen, wie z. B. Lösungen, Syrup und Elixire, lassen sich in Dosierungseinheiten herstellen, so daß eine bestimmte Menge Präparat eine bestimmte Menge Wirkstoff enthält. Syrup kann so hergestellt werden, daß der Wirkstoff in einer wäßrigen Lösung, welche geeignete Geschmacksstoffe enthält, gelöst wird; Elixire werden unter Verwendung nichttoxischer, alkoholischer Trägerstoffe erhalten. Suspensionen kann man durch Dispergieren der Verbindung in einem nicht toxischen Trägerstoff darstellen. Lösungsvermittler und Emulgiermittel, wie

z. B. äthoxylierte Isostearylalkohole und Polyoxyäthylensorbitester, Konservierungsmittel, geschacksverbessernde Zusätze wie z. B. Pfefferminzöl oder Saccharin und dergleichen können auch zugegeben werden.

Dosierungsvorschriften können auf der Kapsel angegeben werden. Überdies kann die Dosierung so abgesichert sein, daß der Wirkstoff verzögert abgegeben wird, z. B. durch Einhalten des Wirkstoffes in Polymerensubstanzen, Wachse oder dergleichen.

Zusätzlich zu den oben erwähnten pharmazeutischen Zusammensetzungen lassen sich auch diese Wirkstoffe enthaltende Lebensmittel hergestellt werden; beispielsweise Zucker, Brot, Kartoffelprodukte, Fruchtsaft, Bier, Schokolade und andere Konfektartikel, und Konserven, wie z. B. Marmelade, wobei zu diesen Produkten eine therapeutisch-wirksame Menge mindestens eines der erfindungsgemäßen Inhibitoren gegeben wurde.

Herstellungsbeispiele

1. N-Methyl-1-desoxy-manno-nojirimycin

6 g (30 mMol) Manno-1-desoxynojirimycin-Hydrochlorid, 7,7 g einer 35 %igen Formalin-Lösung (90 mMol) und 1,9 g (30 mMol) Natriumcyanoborhydrid werden in 150 ml Methanol und 30 ml Wasser gelöst, der pH mit Eisessig auf 4-5 gestellt und 2 1/2 Stunden bei RT gerührt. Anschließend wird der pH mit konzentrierter Salzsäure auf 3-4 eingestellt und eine Stunde unter Rückfluß gekocht. Nach dem Abkühlen wird die Lösung über einen sauren Ionenaustauscher gegeben (Lewatit SP 112, $H^+$-Form, in einer Säule 5 x 40 cm), mit 500 ml Methanol gewaschen und mit 500 ml Methanol/konz. Ammoniak 10:1 eluiert. Nach dem Einrotieren des Eluats wird das Produkt aus Ethanol kristalliniert. Man erhält 5-6 g des gewünschten Produkts.
Fp.: 139°C

2. N-Ethyl-1-desoxy-manno-nojirimycin

Herstellung analog Beispiel 1 mit Acetaldehyd statt Formalin.
MS: $11^+$ = 191

3. N-Propyl-1-desoxy-manno-nojirimycin

Herstellung analog Beispiel 1 mit Propionaldehyd statt Formalin.
MS: $11^+$ = 204

4. N-Heptyl-1-desoxy-manno-nojirimycin

Herstellung analog Beispiel 1 mit Heptanal statt Formalin.
MS: $11^+ = 261$

5. N-Dodecyl-1-desoxy-manno-nojirimycin

Herstellung analog Beispiel 1 mit Dodecanal statt Formalin
MS: $11^+ = 333$

6. N-(3-Phenylpropyl)-1-desoxy-manno-nojirimycin

Herstellung analog Beispiel 1 mit 3-Phenylpropanal statt Formalin.
Fp.: 55°C

7. N-p-chlorbenzyl-1-desoxy-manno-nojirimycin

Herstellung analog Beispiel 1 mit p-Chlorbenzaldehyd statt Formalin.
Fp.: 43°C

8. N-Carboxymethyl-1-desoxy-manno-nojirimycin

20 g (0.1 mol) Manno-1-desoxynojirimycin-Hydrochlorid, 19 g (0.1 mol) Bromessigsäure-tert-butylester, 13,8 g (0.1 mol ) $K_2CO_3$ und 1 g KJ werden über Nacht bei Raumtemperatur in 150 ml Methanol gerührt. Anschließend wird von den Salzen abfiltriert, mit konzentrierter Salzsäure ein pH von

4-5 eingestellt und analog Beispiel 1 über einen sauren Ionenaustauscher (Lewatit SP 112, $H^+$-Form) gegeben. (Anmerkung: der tert.-Butylester wird durch den sauren Ionenaustauscher in die freie Säure überführt).
Fp.: 205°C

9. <u>N-(3-Phenyl-3-propenyl)-1-desoxy-manno-nojirimycin</u>

Herstellung analog Beispiel 8 mit 3-Phenyl-3-propenylbromid statt Bromessigsäure-ter.-butylester.
MS: $M^+$ = 279

## Patentansprüche

1. Verbindungen der Formel (I)

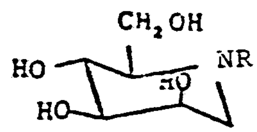

(I)

in der
R einen gegebenenfalls substituierten, geradkettigen, verzweigten oder cyclischen, gesättigten oder ungesättigten aliphatischen Kohlenwasserstoffrest darstellt.

2. Verbindungen gemäß Anspruch 1, dadurch gekennzeichnet, daß R einen Alkylrest mit 1 bis 30 C-Atomen oder einen Alkenylrest oder einen Alkinylrest mit 2 bis 18 C-Atomen darstellt.

3. Verbindungen gemäß Anspruch 1, dadurch gekennzeichnet, daß R einen Alkylrest mit 1 bis 18 C-Atomen oder einen Alkenylrest oder einen Alkinylrest mit 3 bis 10 C-Atomen darstellt.

4. Verbindungen gemäß Anspruch 1, dadurch gekennzeichnet, daß R einen Alkylrest mit 1 bis 12 C-Atomen darstellt.

5. Verbindungen gemäß Anspruch 1, dadurch gekennzeichnet, daß R einen Methyl-, Ethyl-, Propyl-, Heptyl-,

Dodecyl-, Phenoxypropyl-, Halogenbenzyl-, Caroxymethyl- oder Phenylpropenylrest darstellt.

6. Verbindungen gemäß den Ansprüchen 1 bis 5 in Form ihrer pharmazeutisch verwendbaren Salze und in Form ihrer Bio-Vorläufer.

7. Verbindungen gemäß Anspruch 6 in Form ihrer Hydrochloride, -sulfate, -acetate, -carbonate oder -oxalate.

8. Verfahren zur Herstellung von Verbindungen der Formel (I)

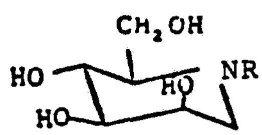

(I)

in der

R einen gegebenenfalls substituierten, geradkettigen, verzweigten oder cyclischen, gesättigten oder ungesättigten aliphatischen Kohlenwasserstoffrest darstellt, dadurch gekennzeichnet, daß man Manno-1-desoxynojirimycin der Formel (II)

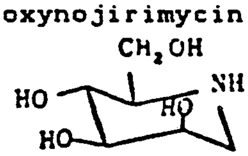

(II)

mit Carbonylverbindungen der Formel (III)

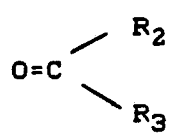

in der
$R^2$ und $R^3$ entweder H bedeuten oder die oben für R angegebene Bedeutung besitzen oder $R^2$ für H und $R^3$ für die oben angegebene Bedeutung von R steht, in Gegenwart eines Wasserstoff-Donor-Reduktionsmittels umsetzt.

9. Verfahren zur Herstellung von Verbindungen der Formel (I)

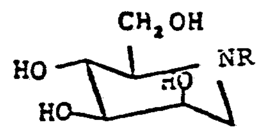

(I)

in der
R einen gegebenenfalls substituierten, geradkettigen, verzweigten oder cyclischen, gesättigten oder ungesättigten aliphatischen Kohlenwasserstoffrest darstellt, dadurch gekennzeichnet, daß man Manno-1-desoxynojirimycin der Formel (II)

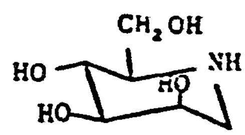

(II)

mit reaktiven Alkylierungsmitteln der Formel (IV)

Z - R (IV)

in der
R die oben angegebene Bedeutung besitzt und Z eine bei Alkylierungsmitteln gebräuchliche leicht austretende Gruppe darstellt, umsetzt und die Reaktionsansätze in üblicher Weise aufarbeitet.

10. Verfahren zur Herstellung von Verbindungen der Formel (I)

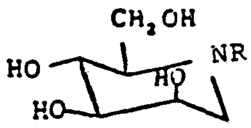

(I)

in der R einen gegebenenfalls substituierten, geradkettigen, verzweigten oder cyclischen, gesättigten oder ungesättigten aliphatischen Kohlenwasserstoff darstellt, dadurch gekennzeichnet, daß man Manno-1-desoxynojirimycin der Formel (II)

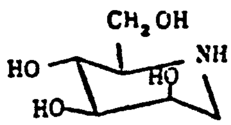

(II)

mit Acylverbindungen der Formel (V)

R-CO-Hal

in der R die oben angegebene Bedeutung besitzt und Hal für Halogen steht, oder mit einem den Rest R enthaltenden Anhydrid umsetzt und die entstehenden Verbindungen anschließend mit einem Reduktionsmittel reduziert.

11. Verbindungen der Formel (I)

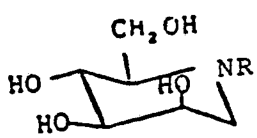

(I)

in der
R einen gegebenenfalls substituierten, geradkettigen, verzweigten oder cyclischen, gesättigten oder ungesättigten aliphatischen Kohlenwasserstoffrest darstellt, zur Anwendung bei der therapeutischen Behandlung des menschlichen oder tierischen Körpers.

12. Verbindungen der Formel (I)

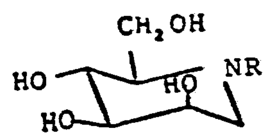
(I)

in der
R einen gegebenenfalls substituierten, geradkettigen, verzweigten oder cyclischen, gesättigten oder ungesättigten aliphatischen Kohlenwasserstoffrest darstellt, zur Verwendung als Mannosidaseinhibitoren.

13. **Arzneimittel** enthaltend Verbindungen der Formel (I)

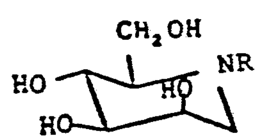
(I)

in der
R einen gegebenenfalls substituierten, geradkettigen, verzweigten oder cyclischen, gesättigten oder ungesättigten aliphatischen Kohlenwasserstoffrest darstellt.

14. Verwendung von Verbindungen der Formel (I)

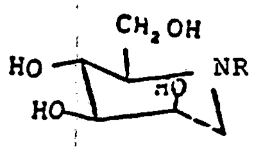

(I)

in der R einen gegebenenfalls substituierten, geradkettigen, verzweigten oder cyclischen, gesättigten oder ungesättigten aliphatischen Kohlenwasserstoffrest darstellt, bei der Herstellung von Arzneimitteln, die einer Bekämpfung von Krankheiten des menschlichen oder tierischen Körpers dienen.